Europäisches Patentamt

(19) European Patent Office

Office européen des brevets

(11) Veröffentlichungsnummer: **0 017 785**
**A2**

(12) **EUROPÄISCHE PATENTANMELDUNG**

(21) Anmeldenummer: 80101481.2

(22) Anmeldetag: 20.03.80

(51) Int. Cl.³: **C 07 G 7/00, A 61 K 37/02**
**// A61K35/52, A61K37/64**

(30) Priorität: 26.03.79 DE 2911868

(43) Veröffentlichungstag der Anmeldung: 29.10.80
Patentblatt 80/22

(84) Benannte Vertragsstaaten: **BE CH FR GB SE**

(71) Anmelder: **Max-Planck-Gesellschaft zur Förderung der Wissenschaften e.V., Bunsenstrasse 10,**
**D-3400 Göttingen (DE)**
Anmelder: **Council of Scientific and Industrial Research, New Delhi (IN)**

(72) Erfinder: **Bhargava, Pushpa Mittra, Regional Research Laboratory, Hyderabad-500009 Andhra Pradesh (IN)**
Erfinder: **Reddy, Ergam Reddy Shyam Prasad, Regional Research Laboratory, Hyderabad-500009 Andhra Pradesh (IN)**
Erfinder: **Scheit, Karl Heinz, Prof. Dr., Max-Planck-Institut Am Fassberg, D-3400 Göttingen (DE)**

(74) Vertreter: **Weickmann, Heinrich, Dipl.-Ing Patentanwälte Dipl.Ing.H.Weickmann et al, Dipl.Phys.Dr.K.Fincke Dipl.Ing.F.A.Weickmann Dipl.Chem.B.Huber, Dr.-Ing.H.Liska Möhlstrasse 22, D-8000 München 86 (DE)**

(54) Seminalplasmin, Verfahren zu seiner Herstellung und seine Verwendung.

㊗ Seminalplasmin ist ein neues Protein, welches durch einen Sedimentationskoeffizienten von 1,04 S in der Ultrazentrifuge, einen isoelektrischen Punkt von 9,8, Fehlen von Methionen als Aminosäurebestandteil charakterisiert ist und durch Abtrennung der Spermatozoen aus Säugetiersperma, Dialyse des so erhaltenen Plasmas gegen eine Pufferlösung, pH 5 bis 9, Behandlung des dialysierten Plasmas mit einem Anionenaustauscher und danach mit einem Kationenaustauscher, Elution des adsorbierten Proteins vom Kationenaustauscher mit einer Pufferlösung und Feinreinigung des so erhaltenen Produkts nach an sich bekannten biochemischen Methoden unter Anwendung der Wachstumshemmung von E. coli als Testsystem für die Reinigung erhältlich ist. Es eignet sich als bakterizides, fungizides oder Antitumormittel oder zur Hemmung der RNA-Synthese.

0017785

- 1 -

Seminalplasmin, Verfahren zu seiner Herstellung und seine Verwendung

Die Erfindung betrifft das neue Protein Seminalplasmin, ein Verfahren zu seiner Herstellung und seine Verwendung.

Die Erfindung beruht auf der überraschenden Auffindung eines neuen, als Seminalplasmin bezeichneten Proteins in Säugetiersperma, welches interessante Eigenschaften aufweist, die seine gewerbliche Verwertung ermöglichen. Insbesondere weist dieses neue Protein antibakterielle, antifungizide und anticancerogene (tumorabtötende) Eigenschaften auf und stellt einen Inhibitor der RNA-Synthese dar.

Säugetiersperma, wie Bullensperma, stellt ein komplexes Gemisch der Sekretionen von verschiedenen Nebendrüsen dar, das zusammen mit den Spermatozoen von dem männlichen Säugetier abgegeben werden kann. Bei der Aufarbeitung des Spermas nach Abtrennung der Spermatozoen wurde das erfindungsgemäße neue Protein aufgefunden und isoliert.

Erfindungsgemäßes gereinigtes Seminalplasmin erwies sich in der Polyacrylamid-Gelelektrophorese sowohl mit als auch ohne Natriumdodecylsulfat als homogen. Ebenfalls homogen erwies es sich bei Sedimentationsversuchen in einer analytischen Ultrazentrifuge, wobei es einen Sedimentationskoeffizienten von 1,o4 S zeigte, sowie bei einer isoelektrischen Fokussierung auf einer Säule (isoelektrischer Punkt 9,8). Bei der Polyacrylamid-Gelelektrophorese mit Natriumdodecylsulfat (SDS-PAGE) unter Verwendung von kalibrierten Gelen ergaben sich je nach der angewendeten Bestimmungsmethode unterschiedliche Resultate. So wurde ein MG 17ooo bei Bestimmung gemäß "Techniques for High Resolution Elextrophoresis" (ORTEC Application Note AN 32A) ORTEC Inc., Oak Ridge, Tennessee, 1973, jedoch nur von 1o6oo erhalten, wenn gemäß Nature 227, 68o bis 685 (197o) gearbeitet wurde. Sedimentationsversuche in der analytischen Ultrazentrifuge deuteten auf ein Molekulargewicht von 8ooo. Das minimale Molekulargewicht, welches aus der Aminosäurezusammensetzung errechnet wurde, betrug 198oo. Dabei wurde davon ausgegangen, daß pro Mol Protein 1 Mol Valin vorhanden ist. Die unterschiedlichen Resultate bei den verschiedenen Methoden zur Molekulargewichtsbestimmung könnten vielleicht auf ein ungewöhnlich hohes Axialverhältnis im Protein zurückzuführen sein.

Die Aminosäurezusammensetzung in Mol pro 1oo Mol Gesamtaminosäuren (ausgenommen Try) wurde wie folgt bestimmt:

Lys 12,3o; Arg 9,6o; His 4,15; Asx 11,26; Glx 4,92; Phe 3,95; Tyr 1,66; Pro 3,13; Cys 2,98; Thr 1,75; Ser 7,2o; Gly 5,75; Ala 7,9o; Val o,63; Leu 14,2o; Ile 1,8o. Das Protein erwies sich als reich an basischen Aminosäuren und enthält kein Methionin.

Seminalplasmin ist ein potenter Inhibitor des Wachstums von Mikroorganismen, und zwar sowohl von gram-positiven Bakterien, gram-negativen Bakterien als auch von Hefen. In flüssigen Kulturen, die auf einem synthetischen Medium wachsen gelassen waren, beginnend mit einem Inokulum von $10^6$ bis $10^7$ Zellen/ml bei Bakterien und $10^5$ bis $10^6$ Zellen/ml bei Hefen hemmt Seminalplasmin beispielsweise das Wachstum von Streptococcus faecalis, E. coli und Cryptococcus neoformans vollständig bei einer Konzentration von 5o bis 1oo ug (2,5 bis 5,5 nmol) bzw. 25 bis 5o ug (1,25 bis 2,5o nmol) bzw. 12,5 bis 25 ug (o,63 bis 1,26 nmol) pro ml. Vergleichbare Hemmung des Mikroorganismuswachstums bei bekannten Mitteln gleicher Wirkungsrichtung in einer flüssigen Konzentration sind in nmol/ml: Penicillin und Streptomycin, 1,7 bis 8,5; Chloramphenicol 9 bis 27; Tetracycline 2,2 bis 11; Erythromycin 1,4 bis 2,8; Bacitracin 4,5 bis 9; Polymyxin o,9 bis 2,2. Ähnliche Aktivität wurde gegenüber Salmonella thyphimurium, Candida albicans, Bacillus subtilis und Staphylococci nachgewiesen. Keine Aktivität wurde bei Pseudomonas und Proteus gefunden.

Seminalplasmin ist bakterizid und nicht bakteriostatisch wirksam. Wird eine Kultur von E. coli W 16o-37 mit einem Gehalt von etwa $10^7$ Zellen/ml mit Seminalplasmin ($\geq$ 4o ug/ml) 7 Stunden bei 37°C inkubiert (wobei die Kontrolle dann 2,3 x $10^6$ Zellen/ml aufweist) und dann o,5 ul der Kultur auf einen Nähragar übertragen wird, so wird gewöhnlich eine vollständige Wachstumshemmung beobachtet. Bei 2o ug/ml Seminalplasmin wurden üblicherweise über 99 % Reduktion an lebensfähigen Zellen festgestellt. Gelegentlich wurde eine 1oo %-ige Verminderung sogar bei dieser Konzentration erhalten.

- 4 -                              0017785

Seminalplasmin ist wärmelabil. Wird Seminalplasmin 1o
Minuten auf 9o$^\circ$C erhitzt und dann in einer Konzentration von 4o μg/ml wachsenden E. coli-Zellen (1o$^7$/ml)
zugesetzt, so betrug die Wachstumshemmung während 6
Stunden etwas über 95 %, während mit nichterhitztem
Seminalplasmin unter gleichen Bedingungen 1oo % Wachstumshemmung erzielt werden. Lyophilisiertes reines Seminalplasmin erwies sich bei 4$^\circ$C als mindestens 2 Jahre
stabil. In wäßriger Lösung beträgt die Stabilität in
gefrorenem Zustand wenigstens 6 Monate.

Bei E. coli W 16o-37 zeigte Seminalplasmin (1oo μg/ml)
keine Wirkung auf die Syntheserate von DNA und Protein.
Dagegen wird die RNA-Synthese gehemmt. Verschiedene
Anzeichen deuten darauf hin, daß es sich hierbei spezifisch um bestimmte RNA-Typen handelt, die der Hemmung unterliegen. Die vorliegenden Resultate lassen annehmen, daß die mRNA-Synthese wahrscheinlich nicht,
die rRNA-Synthese hingegen schon gehemmt wird.

An E. coli durchgeführte Versuche deuten darauf hin,
daß die antimikrobielle Wirksamkeit des Seminalplasmins
auf dieser Inhibierung der rRNA-Synthese beruht. Dabei
scheint das Seminalplasmin seine Wirksamkeit im Zellinneren zu entfalten, also in die Zelle einzudringen,
und nicht an die Zelloberfläche gebunden zu werden.

Ein weiterer Gegenstand der Erfindung ist ein Verfahren
zur Gewinnung des Seminalplasmins aus Säugetiersperma.

Das erfindungsgemäße Verfahren zur Herstellung von Seminalplasmin ist dadurch gekennzeichnet, daß aus Säugetiersperma die Spermatozoen abgetrennt, das Plasma gegen eine Pufferlösung von pH 5 bis 9 dialysiert, das
dialysierte Plasma über einen Anionenaustauscher gege-

0017785

ben und die nicht adsorbiere Fraktion auf einen Kationenaustauscher gegeben und dieser dann mit einer Pufferlösung eluiert wird.

Man erhält auf diese Weise rohes Seminalplasma, welches bereits die oben beschriebene Wirksamkeit zeigt und sich beispielsweise als antimikrobielles Mittel einsetzen läßt. Es enthält jedoch dann noch eine Ribonuclease, die durch Feinreinigung abgetrennt werden kann, falls sie stören sollte.

Die weitere Reinigung des Seminalplasmins läßt sich nach üblichen biochemischen Methoden durchführen unter Anwendung des Bakterienhemmtests, vorzugsweise unter Anwendung von E. coli.

Die Feinreinigung erfolgt vorzugsweise derart, daß das vom Kationenaustauscher eluierte rohe Seminalplasma über einem Molekularsieb und einer Affinitätschromatographiesäule, besonders bevorzugt über $\underline{/}5'$-(p-Aminophenylphosphoryl)-uridin-2'(3')-phospha$\underline{t}$/-agarose gereinigt wird.

Die Verfolgung der Anreicherung des Seminalplasmins durch den Wachstumshemmtest bei E. coli ergab im Konzentrationsbereich von 1 bis 2o/ug Seminalplasmin pro ml, daß die prozentuale Hemmung des Kulturwachstums bei einem Gehalt von etwa $10^7$ Zellen/ml ($A_{760}$ o,o1) dem Logarithmus der Konzentration proportional ist.

Die durchschnittliche Ausbeute an gereinigtem Seminalplasmin betrug 18 mg/1oo ml Samen, entsprechend einer Ausbeute von etwa 5o %. Das nach der Feinreinigung durch Affinitätschromatographie erhaltene Seminalplasmin ist frei von allen feststellbaren RNAse-, DNAse-oder Protease-Aktivitäten. Es weist die oben erwähnten Eigenschaften bei der Bestimmung des Molekulargewichts nach den verschiedenen Methoden und bezüglich der Aminosäurezusammensetzung auf.

Gereinigtes Seminalplasmin inhibiert beispielsweise das Wachstum von Zajdela aszitischen Hepatomazellen (eine Art von Rattenleberkrebszellen) sowohl in vivo als auch in vitro. In vitro beträgt die Hemmkonzentration dabei etwa 2 bis 5 ug/ml. Für die in-vivo-Inhibierung sind größere Mengen erforderlich.

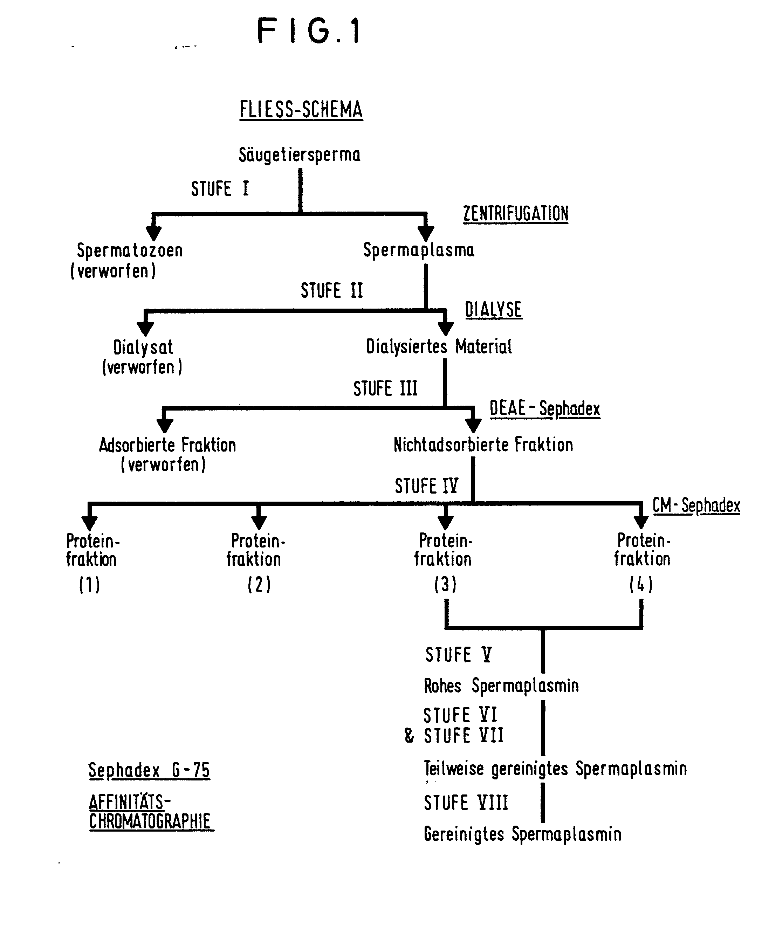

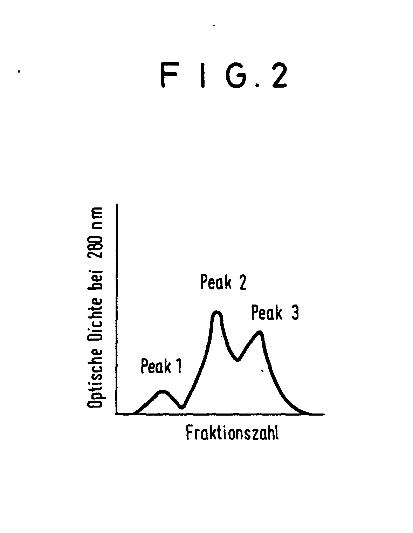

Die Gewinnung von rohem und gereinigtem Seminalplasmin wird im folgenden anhand einer bevorzugten Ausführungsform näher erläutert. Das Verfahren zu seiner Gewinnung wird in dem beigefügten Fließschema der Fig. 1 dargestellt. In Fig. 2 ist eine typische Trennung während der vorletzten Reinigungsstufe dargestellt, wobei die Peaks 1, 2 und 3 drei unterschiedliche Fraktionen des Proteins darstellen.

Stufe I

Säugetiersperma wird in seine Komponenten Spermaplasma und Spermatozoen getrennt, beispielsweise durch Zentrifugieren bei 5oo bis 7oo g während einer Zeit im Bereich von 5 bis 15 Minuten bei einer Temperatur zwischen 9 bis 4°C und erneutem Zentrifugieren der obenstehenden Lösung (Spermaplasma) bei 12oo bis 16oo g während unterschiedlicher Zeiten von 2o bis 6o Minuten.

Stufe II

Die nach I erhaltene überstehende Lösung wird gegen einen Puffer, wie z. B. Tris-HCl-Puffer bei einem pH-Wert zwischen 5 und 9, vorzugsweise 6,o und 8,o, dialysiert.

## Stufe III

Das dialysierte Spermaplasma von der Stufe II wird auf einen Anionenaustauscher, vorzugsweise einen schwach basischen, wie einen Diäthylaminoäthylgruppen aufweisenden Austauscher auf Kohlenhydratbasis, z. B. DEAE-Sephadex von Pharmacia, Schweden, aufgebracht.

## Stufe IV

Die vom Anionenaustauscher nicht adsorbierte Proteinfraktion wird auf einen Kationenaustauscher, vorzugsweise einen schwach sauren, wie einen Carboxymethylgruppen aufweisenden Austauscher auf Kohlenhydratbasis, z. B. CM-Sephadex der Pharmacia, Schweden, aufgebracht. Das adsorbierte Protein wird aus dem Kationenaustauscher unter Verwendung eines Puffers, wie Tris-HCl-Puffer und mit einem ionischen Gradienten, wie dem von Natriumchlorid, der von o bis o,8 M variiert wird, eluiert. Man erhält danach 3 bis 4 Peaks (d. h. Proteinfraktionen).

## Stufe V

Die einzelnen Eluatfraktionen, von denen die dritte und vierte (sofern sie erhalten wird) Proteinfraktionen sind, werden gesammelt, dialysiert und lyophilisiert. Das erhaltene Produkt wird als "rohes Seminalplasmin" bezeichnet und kann als solches bereits für viele Zwecke verwendet werden.

Das rohe Seminalplasmin wird gegebenenfalls durch an sich bekannte Reinigungsverfahren, wie Gelfiltration, weiter gereinigt.  Eine derartige Feinreinigung erfolgt vorzugsweise wie nachstehend beschrieben.

Stufe VI

Rohes Seminalplasmin wird auf eine Säule aus vernetztem Dextran ("Sephadex G-75" der Pharmacia Co.) aufgetragen. Ein typisches Trennmuster ist in der Fig. 2 dargestellt.

Stufe VII

Die Fraktionen des letzten Peak, bei der Stufe VI erhalten, werden vereinigt, dialysiert und lyophilisiert.

Stufe VIII

Das bei der Stufe VII (oder V) erhaltene lyophilisierte Produkt wird weiter bis zur Homogenität unter Anwendung der Polyacrylamid-Gelelektrophorese oder/und der Affinitätschromatographie gereinigt. Für die Affinitätschromatographie erwies sich /5'-(p-Aminophenylphosphoryl)-uridin-2'(3')-phospha̱t̲/-agarose als besonders geeignet. Aber auch andere Affinitätschromatographiematerialien sind geeignet.

Wie bereits erwähnt, kann Seminalplasmin in gereinigter oder in roher Form als antimikrobielles Mittel sowohl bei Bakterien als auch bei Fungi verwendet werden. Desgleichen kommt der neuen Substanz eine ausgeprägte Antitumorwirkung zu. Ferner kann rohes oder gereinigtes Seminalplasmin als Inhibitor der Transkription sowohl in vivo als auch in vitro eingesetzt werden, da es spezifisch die RNA-Synthese hemmt. Die Inhibierung der Transkription wird bei einer Vielzahl von Organismen in vivo und in einer Vielzahl von Systemen in vitro, bei denen die DNA-Template (entweder natürliche oder synthetische) und die RNA-Polymerasen (beispielsweise E.coli-RNA-polymerase, tierische RNA-Polymerasen, Pflanzen-RNA-polymerasen) variiert werden, nachgewiesen. Nachstehende Tabelle zeigt einige Ergebnisse derartiger Versuche über die Transkriptionshemmung.

<u>TABELLE</u>

Inhibierung der Transkription durch Seminalplasmin in vitro

| Templat | Konzentration an Seminalplasmin, die für eine 5o %-ige Inhibierung erforderlich ist ($\mu$g/ml) |
| --- | --- |
| $T_7$-DNA | 25 |
| Kalbsthymus-DNA | 25 |
| Poly dA | 5o |
| Poly dT | 5o |
| Poly dA.Poly dA | 3o |
| Poly d(A-T) | 5o |
| Poly d(G-C) | 1oo |
| Poly d(I-C) | 1oo |

Die Wirkung des Seminalplasmins auf die RNA-Synthese und die Transkription beruht nach den Ergebnissen der durchgeführten Untersuchungen auf einer starken Bindung an die Polymerase. Seminalplasmin stellt damit den ersten Proteininhibitor eukariotischen Ursprungs dar, welcher RNA-Polymerase zu hemmen vermag.

Seminalplasmin ist das erste antimikrobielle Protein, welches in die intakte bakterielle Zelle eindringt. Ebenso ist es der erste bekannte Inhibitor, welcher die Transkription von rRNA in der Ganzzelle spezifisch inhibiert.

Das folgende Beispiel erläutert die Gewinnung und Reinigung des Seminalplasmins weiter.

0017785

B e i s p i e l

Mit Hilfe einer künstlichen Vagina gesammeltes Bullensperma wird bei 6oo g einige Minuten (beispielsweise 1o
bis 15 Minuten) bei 4$^O$C zentrifugiert. Die obenstehende
Lösung (Spermaplasma) wird schließlich bei 14oo g während 3o Minuten bei 4$^O$C zentrifugiert. Die obenstehende Lösung wird in ein Dialysebad gegeben und gegenüber
Tris-Puffer mit einem pH-Wert von etwa 7,4 dialysiert.

Das dialysierte Produkt wird dann auf einen schwach alkalischen Anionenaustauscher (z. B. "DEAE-Sephadex" von
Pharmacia, Schweden) gegeben. Die nichtadsorbierte
Fraktion von der Säule des Anionenaustauschers wird
auf einen schwach sauren Kationenaustauscher (z. B.
"CM-Sephadex" von Pharmacia, Schweden) gegeben. Das
an dem Kationenaustauscher adsorbierte Proteinmaterial
wird unter Verwendung eines Ionengradienten von o bis
o,8 M Natriumchlorid in Tris-HCl-Puffer bei pH 7,4
eluiert. Die den letzten Peak enthaltenden Fraktionen
werden gesammelt, gegenüber Wasser dialysiert und unter Bildung von rohem Seminalplasmin lyophilisiert.

Rohes Seminalplasmin wird durch Gelfiltration (beispielsweise an "Sephadex G-75" der Pharmacia, Schweden) gereinigt. Die aktiven Fraktionen (d. h. solche,
die eine Inhibierung des Wachstums von E. coli zeigen;
im Falle von Sephadex G-75 die Fraktionen, die den
letzten Peak enthalten) werden vereinigt, gegenüber
Wasser dialysiert und lyophilisiert. Das lyophiliserte Material wird weiter bis zur Homogenität durch Polyacrylamid-Gelelektrophorese und durch Chromatographie
an einer Affinitätschromatographiesäule (vorzugsweise
einer Säule aus Agarose-5-(p-aminophenylphosphoryl-uri-
din-2'(3')-phosphat) gereinigt.

- 1 -

Patentansprüche

1.    Seminalplasmin, g e k e n n z e i c h n e t
durch einen Sedimentationskoeffizienten von 1,o4 S in
der Ultrazentrifuge, einen isoelektrischen Punkt von
9,8, Fehlen von Methionin als Aminosäurebestandteil,
erhältlich durch Abtrennung der Speramtozoen aus Säugetiersperma, Dialyse des so erhaltenen Plasmas gegen
eine Pufferlösung, pH 5 bis 9, Behandlung des dialysierten Plasmas mit einem Anionenaustauscher und danach mit einem Kationenaustauscher, Elution des adsorbierten Proteins vom Kationenaustauscher mit einer
Pufferlösung und Feinreinigung des so erhaltenen Produkts nach an sich bekannten biochemischen Methoden
unter Anwendung der Wachstumshemmung von E. coli als
Testsystem für die Reinigung.

2.    Verfahren zur Herstellung von Seminalplasmin
nach Anspruch 1, dadurch g e k e n n z e i c h n e t ,
daß aus Säugetiersperma die Spermatozoen abgetrennt,
das Plasma gegen eine Pufferlösung von pH 5 bis 9 dialysiert, das dialysierte Plasma über einen Anionenaustauscher gegeben und die nicht adsorbierte Fraktion nacheinander auf einem Kationenaustauscher, einem Moleku-

larsiebgel und einer /5'-(p-Aminophenylphosphoryl)-uridin—2'(3')-phospha_t7-agarose Affinitätschromatographiesäule gereinigt wird.

3. Verfahren nach Anspruch 2, dadurch g e k e n n z e i c h n e t , daß die Spermatozoen abzentrifugiert, der Überstand gegen Tris-HCl-Puffer o,oo1 bis o,1 M, pH 6 bis 8, dialysiert und auf einen mit dem gleichen Puffer äquilibrierten Anionenaustauscher gegeben wird.

4. Verfahren nach Anspruch 2 oder 3, dadurch g e k e n n z e i c h n e t , daß ein schwach saurer Kationenaustauscher verwendet und das adsorbierte Protein mit einem Ionengradienten von o bis o,8 M eluiert wird.

5. Verfahren nach einem der Ansprüche 2 bis 4, dadurch g e k e n n z e i c h n e t , daß das Eluat des Kationenaustauschers mit einem Molekularsieb auf Basis von vernetztem Dextran behandelt wird.

6. Verfahren nach einem der Ansprüche 2 bis 5, dadurch g e k e n n z e i c h n e t , daß zur Affinitätschromatographie /5'-(p-Aminophenylphosphoryl)-uridin-2'(3')-phospha_t7-agarose verwendet wird.

7. Verwendung von Seminalplasmin als bakterizides, fungizides oder Antitumormittel oder zur Hemmung der RNA-Synthese.

# FIG.1

FLIESS-SCHEMA

Säugetiersperma

STUFE I

ZENTRIFUGATION

Spermatozoen          Spermaplasma
(verworfen)

STUFE II

DIALYSE

Dialysat          Dialysiertes Material
(verworfen)

STUFE III

DEAE-Sephadex

Adsorbierte Fraktion          Nichtadsorbierte Fraktion
(verworfen)

STUFE IV

CM-Sephadex

Protein-        Protein-        Protein-        Proteinfraktion        fraktion        fraktion        fraktion
(1)             (2)             (3)             (4)

STUFE V

Rohes Spermaplasmin

STUFE VI
& STUFE VII

Sephadex G-75          Teilweise gereinigtes Spermaplasmin

AFFINITÄTS-          STUFE VIII
CHROMATOGRAPHIE
Gereinigtes Spermaplasmin

F I G . 2

Peak 2

Peak 3

Peak 1

Optische Dichte bei 280 nm

Fraktionszahl